# EUROPEAN PATENT APPLICATION

(11) **EP 4 813 501 A1**
(43) Date of publication of application: **30.09.2026**
(21) Application number: 26162442.3
(22) Date of filing: 04.03.2026
(51) Int. Cl.: B01D 53/047, A61M 16/10, B01D 53/04

(54) **PORTABLE OXYGEN SUPPLY SYSTEM**

(30) Priority: 10.03.2025 US 202563769427 P; 03.03.2026 US 202619555524
(71) Applicant: Oxus America, Inc., Orion Township, MI 48326 (US)
(72) Inventor: Thompson, Loren M., Lapeer, MI (US); Abusamra, Gary, Rochester Hills, MI (US); Voto, Andrew, Waterford, MI (US)
(74) Representative: Forresters IP LLP

(57) **Abstract**

An oxygen supply system may deliver oxygen from a first source, such as a removable and refillable oxygen tank, from a second source, such as an oxygen concentrator, or from the first source and the second source in combination.

## Description

### CROSS-REFERENCE TO RELATED APPLICATION(S)

This application claims the benefit of United States Provisional Application No. 63/769,427, filed on March 10, 2025, the content of which is hereby incorporated in its entirety herein for all purposes.

### TECHNICAL FIELD

The present disclosure relates to a portable oxygen supply system.

### BACKGROUND

Oxygen may be supplied from a pre-filled tank or from an oxygen concentrator system.

Oxygen concentrator systems generate substantially pure (e.g., 82-96% pure) oxygen gas using ambient air as an input gas. Oxygen concentrator systems are commonly used for medical applications by patients who have a need for substantially pure oxygen. Oxygen concentrator systems include portable systems that can be carried by patients, and non-portable systems that are often used in a medical facility or in a residential setting. Some known oxygen concentrator systems operate on a pressure swing adsorption or vacuum pressure swing adsorption cycle. In such systems, ambient air is pressurized in a sieve bed of an adsorbent material that adsorbs nitrogen (e.g., zeolite), and substantially pure oxygen is then released from the sieve bed while the nitrogen is retained by the adsorbent material. The nitrogen may then be purged from the adsorbent material.

### SUMMARY

An aspect of the disclosure is a portable multiple-source oxygen supply system. The system includes a housing. The system also includes a portable oxygen concentrator located in the housing and configured to receive ambient air and generate substantially pure oxygen as a product gas, the portable oxygen concentrator including at least one media bed containing an adsorbent material configured to separate oxygen from other components of the ambient air, a compressor configured to pressurize the ambient air, and a valve assembly configured to regulate flow of the ambient air and the product gas. The system also includes an oxygen supply tank configured to store pressurized oxygen, wherein the oxygen supply tank is removably coupled to the housing, and the oxygen supply tank is refillable and replaceable. The system also includes a manifold located in the housing and in fluid communication with the portable oxygen concentrator and the oxygen supply tank, the manifold configured to selectively route oxygen from the portable oxygen concentrator and the oxygen supply tank to a supply port. The system also includes a controller operably located in the housing and electrically connected to the manifold and the portable oxygen concentrator, the controller configured to control operation of the portable oxygen concentrator to generate the product gas, and to control the manifold to selectively provide oxygen to the supply port from the portable oxygen concentrator, from the oxygen supply tank, or from both simultaneously.

In some implementations of the portable multiple-source oxygen supply system, the oxygen supply tank includes a pressure vessel configured to store the pressurized oxygen, and a tank-side coupler configured to selectively connect and disconnect the oxygen supply tank from the manifold using a system-side coupler.

In some implementations of the portable multiple-source oxygen supply system, the manifold includes at least one electrically operated valve operable between a first state, routing oxygen from the oxygen supply tank to the supply port, and a second state, routing oxygen from the portable oxygen concentrator to the supply port.

In some implementations of the portable multiple-source oxygen supply system, the controller is configured to detect depletion of oxygen in the oxygen supply tank and automatically switch the manifold to deliver oxygen solely from the portable oxygen concentrator.

In some implementations of the portable multiple-source oxygen supply system, the manifold further includes a combined supply mode configured to mix oxygen from the portable oxygen concentrator with oxygen from the oxygen supply tank to achieve an increased flow rate at the supply port.

In some implementations of the portable multiple-source oxygen supply system, the system further includes a power source that provides electrical power to the portable oxygen concentrator, the controller, and the manifold.

In some implementations of the portable multiple-source oxygen supply system, the controller is further configured to allow a user to indicate a selected oxygen flow rate through an interface device, and the controller adjusts operation of the portable oxygen concentrator and the manifold based on the selected oxygen flow rate.

In some implementations of the portable multiple-source oxygen supply system, the portable oxygen concentrator operates according to at least one of a pressure swing adsorption cycle or a vacuum pressure swing adsorption cycle using the media bed for nitrogen adsorption.

In some implementations of the portable multiple-source oxygen supply system, an interface device provides user-accessible controls for activating the portable oxygen concentrator, selecting flow rates, and displaying system status.

In some implementations of the portable multiple-source oxygen supply system, the oxygen supply tank is configured to provide oxygen at a first flow rate for a first time period, the portable oxygen concentrator is configured to provide oxygen at a second flow rate for a second time period, the first flow rate is higher than the second flow rate, and the second time period is longer than the first time period.

In some implementations of the portable multiple-source oxygen supply system, the controller is further configured to monitor pressure levels in both the portable oxygen concentrator and the oxygen supply tank to generate an alert when the oxygen supply tank requires replacement or refilling.

In some implementations of the portable multiple-source oxygen supply system, the oxygen supply tank is located external to the housing.

A second aspect of the disclosure is a portable oxygen delivery apparatus. The portable oxygen delivery apparatus according to the second aspect of the disclosure comprises a housing defining an interior space, an oxygen concentrator disposed within the interior space of the housing, a removable oxygen supply tank coupled to an exterior of the housing, a manifold disposed within the interior space, a controller disposed within the interior space, and an interface device communicatively coupled to the controller and accessible from an exterior of the housing. The oxygen concentrator comprises a compressor, at least one media bed containing an adsorbent material, and a valve assembly, and the oxygen concentrator is configured to generate an oxygen-enriched product gas from ambient air drawn through an inlet. The removable oxygen supply tank comprises a pressure vessel configured to store pressurized oxygen and a tank-side coupler configured to selectively establish and break a pneumatic connection with a system-side coupler of the portable oxygen delivery apparatus. The manifold is in fluid communication with both the oxygen concentrator and the removable oxygen supply tank and comprises at least one electrically operated valve configurable in a first state, a second state, and a combined state, wherein oxygen is routed from the removable oxygen supply tank to a supply port in the first state, oxygen is routed from the oxygen concentrator to the supply port in the second state, and oxygen from the oxygen concentrator and the removable oxygen supply tank is simultaneously routed to the supply port in the combined state to achieve a flow rate at the supply port that is greater than a maximum flow rate of the oxygen concentrator alone. The controller is electrically connected to the manifold and the oxygen concentrator and is configured to select among the first state, the second state, and the combined state based on at least one of a user-indicated flow rate or a detected pressure level in the removable oxygen supply tank. The interface device is configured to receive user inputs and display operational status information.

In some implementations of the portable oxygen delivery apparatus according to the second aspect, the controller is further configured to monitor a pressure level within the removable oxygen supply tank using at least one pressure sensor and automatically transition the manifold from the first state or the combined state to the second state upon detecting that the pressure level within the removable oxygen supply tank falls below a threshold value corresponding to depletion of the removable oxygen supply tank.

In some implementations of the portable oxygen delivery apparatus according to the second aspect, the portable oxygen delivery apparatus further comprises a power source disposed within the interior space and configured to provide electrical power to the oxygen concentrator, the manifold, the controller, and the interface device, wherein the power source comprises at least one rechargeable battery.

In some implementations of the portable oxygen delivery apparatus according to the second aspect, the removable oxygen supply tank is configured to supply oxygen at a first flow rate for a first time period, the oxygen concentrator is configured to supply oxygen at a second flow rate for a second time period that is longer than the first time period, wherein the second flow rate is lower than the first flow rate, and in the combined state, the manifold is configured to deliver oxygen from both the removable oxygen supply tank and the oxygen concentrator simultaneously such that a combined flow rate at the supply port is greater than the second flow rate alone.

A third aspect of the disclosure is a method of operating a portable multiple-source oxygen supply system. The method according to the third aspect of the disclosure comprises receiving ambient air at a portable oxygen concentrator housed within a housing, generating by the portable oxygen concentrator a product gas comprising substantially pure oxygen by separating oxygen from other components of the ambient air using at least one media bed containing an adsorbent material, providing pressurized oxygen in an oxygen supply tank that is removably coupled to the housing, controlling by a controller a manifold in fluid communication with the portable oxygen concentrator and the oxygen supply tank to selectively route oxygen to a supply port from the portable oxygen concentrator only, from the oxygen supply tank only, or from the portable oxygen concentrator and the oxygen supply tank simultaneously, and detecting by the controller a depletion condition of the oxygen supply tank and automatically switching the manifold to deliver oxygen solely from the portable oxygen concentrator in response to detection of the depletion condition.

In some implementations of the method according to the third aspect, the method further comprises receiving by the controller a user-selected oxygen flow rate through an interface device and adjusting by the controller operation of the portable oxygen concentrator and the manifold based on the user-selected oxygen flow rate, including transitioning to supply from at least the oxygen supply tank when the user-selected oxygen flow rate exceeds a maximum flow rate achievable by the portable oxygen concentrator alone.

In some implementations of the method according to the third aspect, the method further comprises monitoring by the controller a pressure level within the oxygen supply tank using at least one pressure sensor and generating an alert through an interface device when the pressure level falls below a predetermined threshold value indicating that the oxygen supply tank requires at least one of replacement or refilling.

In some implementations of the method according to the third aspect, generating the product gas comprises operating the portable oxygen concentrator according to at least one of a pressure swing adsorption cycle or a vacuum pressure swing adsorption cycle, wherein nitrogen is adsorbed by the adsorbent material during pressurization of the at least one media bed and purged from the at least one media bed through a vent during a purge phase.

### BRIEF DESCRIPTION OF THE DRAWINGS

The disclosure is best understood from the following detailed description when read, by way of example only, in conjunction with the accompanying drawings. It is emphasized that, according to common practice, the various features of the drawings are not to scale. On the contrary, the dimensions of the various features are arbitrarily expanded or reduced for clarity.
FIG. 1 is a block diagram showing a portable oxygen supply system according to an example.
FIG. 2 is a block diagram of an oxygen concentrator of the portable oxygen supply system.
FIG. 3 is a graph that shows oxygen flow rates over time in a first scenario, a second scenario, and a third scenario.
FIG. 4 is a flowchart of a process of operating a portable multiple-source oxygen supply system.

### DETAILED DESCRIPTION

The disclosure herein relates to a portable multiple-source oxygen supply system. The system includes a portable oxygen concentrator, an oxygen supply tank, a manifold, and a controller. The controller is configured to control operation of the portable oxygen concentrator and is configured to control operation of the manifold. The manifold is configured to regulate the flow of oxygen from the portable oxygen concentrator and the oxygen supply tank to a supply port. The disclosure also relates to a method of operating a portable multiple-source oxygen supply system, the method comprising controlling the supply of oxygen from one or more of the portable oxygen concentrator and the oxygen supply tank to a supply port.

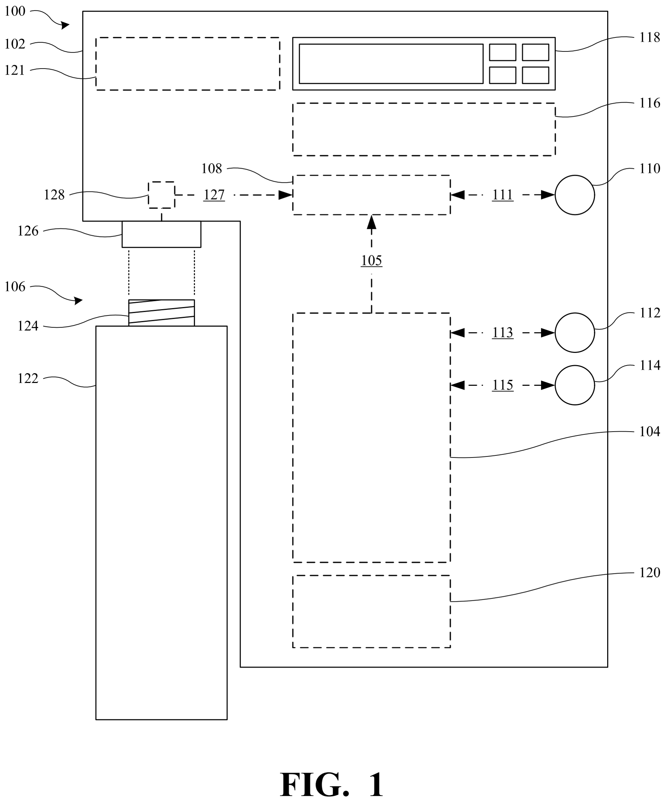

FIG. 1 is a block diagram of a portable multiple-source oxygen supply system, which is referred to herein as a system 100. The system 100 includes a housing 102, an oxygen concentrator 104, an oxygen supply tank 106, a manifold 108, a supply port 110, an inlet 112, a vent 114, a controller 116, an interface device 118, and a power source 120. The system 100 is a multi-source oxygen supply system, meaning that it can supply oxygen from the oxygen concentrator 104, from the oxygen supply tank 106, or from the oxygen concentrator 104 and the oxygen supply tank 106 in combination. While a portable system is described herein, it should be understood that some or all of the features of the system 100 could be incorporated in a non-portable system. The system 100 may also be referred to herein as a portable oxygen delivery apparatus, and the terms "system" and "apparatus" are used interchangeably throughout this disclosure to refer to the same device.

The housing 102 is a structure that other components of the system 100 are contained in or coupled to. As an example, the oxygen concentrator 104, the manifold 108, the controller 116, and the power source 120 may be located within an interior space defined by the housing 102. As an example, the oxygen supply tank 106, the supply port 110, the inlet 112, the vent 114, and the interface device 118 may be coupled or couplable to the housing 102 at the exterior thereof, such that they are accessible from the exterior of the housing 102. Thus, the oxygen supply tank 106 may be located external to the housing 102. The housing 102 may be a generally rigid structure, for example, formed from plastic, metal, another suitable material, or combinations thereof. The housing 102 may be a single-part structure or may be a multi-part structure.

The oxygen concentrator 104 may be configured to generate substantially pure oxygen. For example, using a supply of ambient air as an input, non-oxygen components of the ambient air (e.g., nitrogen) may be removed, resulting in substantially pure oxygen as a product gas. The oxygen concentrator 104 will be described further herein. To deliver the product gas (e.g., to a patient via a cannula, or to another usage), the oxygen concentrator 104 is pneumatically connected to the manifold 108 by a concentrator line 105. The concentrator line 105 is a pneumatic line that allows gas from the oxygen concentrator 104 to be provided to the manifold 108. Through the manifold 108, the gas from the oxygen concentrator 104 may be delivered to the supply port 110 for use.

The oxygen supply tank 106 is configured to store pressurized oxygen, such as substantially pure oxygen. The oxygen supply tank 106 is removable from the system 100 and is refillable. The oxygen supply tank 106 may also be referred to herein as a removable oxygen supply tank, as it is configured to be removed from and reattached to the system 100. The removable oxygen supply tank comprises a pressure vessel 122 and a tank-side coupler 124. The pressure vessel 122 contains the gas. The volume of the pressure vessel 122 may vary based on the application, such as one hundred and fifty liters in an example. The gas is stored under pressure. As an example, the gas may be stored at pressures such as at 150 bar - 300 bar (e.g., 15,000 kPa - 30,000 kPa), but other pressures may be used. The tank-side coupler 124 allows the gas to be provided to the pressure vessel 122 during filling and allows the gas to be expelled from the pressure vessel 122 during usage. The removable oxygen supply tank is accessible from an exterior of the system 100 and is configured to allow removal of the removable oxygen supply tank and replacement thereof with another tank, thereby allowing the removable oxygen supply tank to be filled by a separate system and allowing for use of multiple tanks with the system 100.

The tank-side coupler 124 connects to a system-side coupler 126 of the system 100. The system-side coupler 126 is in fluid communication with the manifold 108 through a tank line 127. The tank line 127 is a pneumatic line that allows gas from the oxygen supply tank 106 to be provided to the manifold 108. Through the manifold 108, the gas from the oxygen supply tank 106 may be delivered to the supply port 110 for use. The at least one pressure sensor 128 may be located on the tank line 127 to allow fluid communication between the at least one pressure sensor 128 and the oxygen supply tank 106 for sensing of the pressure in the oxygen supply tank 106.

The tank-side coupler 124 allows the oxygen supply tank 106 to be selectively connected to and disconnected from the other portions of the system 100. This may be referred to as moving the oxygen supply tank 106 between a connected position with respect to the system 100, and a disconnected position with respect to the system 100. The tank-side coupler 124 is configured to selectively establish and break the pneumatic connection between the oxygen supply tank 106 and the system-side coupler 126, thereby allowing the oxygen supply tank 106 to be connected and disconnected from the system 100 as needed. When in the connected position, gas may flow from the oxygen supply tank 106 to the system 100. As is conventional, the tank-side coupler 124 may incorporate a check valve to retain the gas in the pressure vessel 122 when the oxygen supply tank 106 is in the disconnected position. The tank-side coupler 124 and the system-side coupler 126 are configured to establish both a pneumatic connection and a mechanical connection between the oxygen supply tank 106 and the system 100. As examples, the tank-side coupler 124 and the system-side coupler 126 may be coupled by a threaded connection, a quick-release connection, or another suitable type of connection. In use, pressurized oxygen is provided from the removable oxygen supply tank to the manifold 108 and subsequently to the supply port 110. The removable oxygen supply tank may arrive pre-filled with pressurized oxygen, or may be refilled and then connected to the system 100 prior to use.

The manifold 108 is controllable to change how gas flows through the system 100. To allow the manifold 108 to be controlled, it may include one or more electrically operated valves. Thus, as one example, the manifold 108 can be controlled to cause it to transmit the product gas from the oxygen concentrator 104 to the supply port 110, as will be described. In a first operating state of the manifold 108, the valves of the manifold 108 connect the tank line 127 to the supply line 111 to allow oxygen from the oxygen supply tank 106 to be provided to the supply port 110 for use. In a second operating state of the manifold 108, the valves of the manifold 108 connect the concentrator line 105 to the supply line 111 to allow oxygen from the oxygen concentrator 104 to be provided to the supply port 110 for use. Thus, the manifold 108 may comprise at least one electrically operated valve operable between a first state routing oxygen from the oxygen supply tank 106 to the supply port and a second state routing oxygen from the oxygen concentrator 104 to the supply port. In some implementations, the manifold 108 is further configurable in a combined state (e.g., a combined supply mode) in which oxygen from both the oxygen concentrator 104 and the oxygen supply tank 106 is simultaneously routed to the supply port 110. In the combined state, the manifold 108 is configured to mix oxygen from the oxygen concentrator 104 with oxygen from the oxygen supply tank 106 to achieve an increased combined flow rate at the supply port 110, the combined flow rate being greater than a maximum flow rate achievable by the oxygen concentrator 104 alone. Thus, the manifold 108 may be configured to selectively route oxygen from the oxygen concentrator 104 and the oxygen supply tank 106 to the supply port 110.

To provide a source of input gas (e.g., ambient air) for use by the oxygen concentrator 104, the oxygen concentrator 104 is coupled to the inlet 112 of the system 100 by an inlet line 113. The inlet 112 may be located on the exterior of the system 100 (e.g., as an opening defined by the housing 102) and is in communication with ambient air external to the system 100. Thus, ambient air may be introduced into (e.g., drawn into) the system 100 through the inlet 112 for use by the oxygen concentrator 104 to generate the product gas.

To allow venting from the oxygen concentrator 104, the oxygen concentrator 104 is coupled to the vent 114 by a vent line 115. The vent 114 may be in communication with ambient air external to the system 100. Venting is used to remove gases from the oxygen concentrator 104 when the gases are not intended for use. As an example, nitrogen may be occasionally purged to the ambient environment using the vent line 115 and the vent 114.

Operation of the oxygen concentrator 104 may be regulated by the controller 116 (e.g., according to program instructions) and may be controlled in response to user inputs made through the interface device 118. The interface device 118 may include, for example, buttons, dials, indicator lights, a display screen, and/or other components that facilitate input and output functions. In some implementations, the interface device 118 provides user-accessible controls for activating the oxygen concentrator 104, selecting flow rates, and displaying system status. The interface device 118 may be configured to receive user inputs and to display operational status information regarding the system 100, such as current oxygen flow rate, remaining oxygen supply in the oxygen supply tank 106, operating mode of the manifold 108, and alert notifications. Additional components may be included in the oxygen concentrator 104, including conventional components as are known in the art.

The power source 120 provides power to components of the system 100, including the oxygen concentrator 104, the manifold 108, the controller 116, and the interface device 118. The power source 120 is located within the interior space defined by the housing 102. In some implementations, the power source 120 includes at least one rechargeable battery, such as rechargeable batteries 121, disposed within the interior space of the housing 102. The at least one rechargeable battery may be of any suitable type, such as a lithium-ion or lithium-polymer rechargeable battery. In some implementations, the power source 120 may additionally or alternatively include a connection to an external power supply, such as a standard electrical outlet or a vehicle power adapter, allowing the system 100 to operate on external power and, in some implementations, to recharge the at least one rechargeable battery while in operation. Thus, in some implementations, the system 100 includes the power source 120 comprising at least one rechargeable battery, which provides electrical power to the oxygen concentrator 104, the controller 116, the manifold 108, and the interface device 118, the power source 120 being located within the interior space of the housing 102.

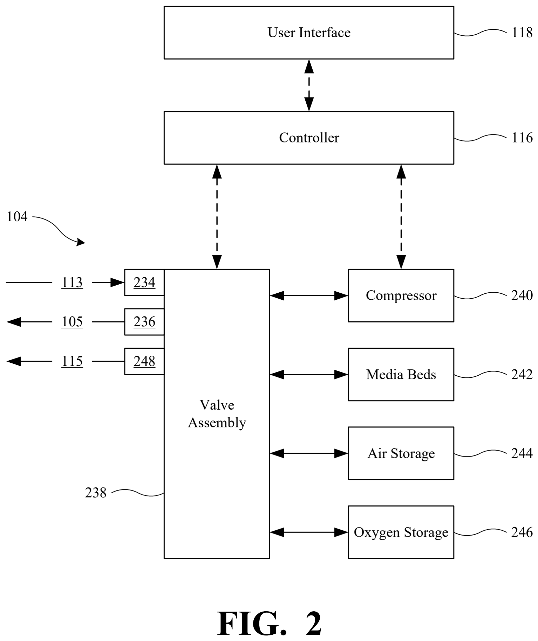

FIG. 2 is a block diagram of the oxygen concentrator 104, showing components thereof as well as electrical and pneumatic connections between such components. The oxygen concentrator 104 may be an oxygen concentration device of any kind, such as one that operates on a pressure swing adsorption (PSA) cycle or a vacuum pressure swing adsorption (VPSA) cycle. The oxygen concentrator 104 is operable to receive air as an input gas and separate the air into constituent components to generate a product gas and an output. As an example, the oxygen concentrator 104 may be configured to generate an oxygen-enriched product gas, the oxygen-enriched product gas comprising at least 50% oxygen. As another example, the oxygen concentrator 104 may be configured to generate substantially pure oxygen as the product gas, the substantially pure oxygen comprising 82-96% oxygen.

The oxygen concentrator 104 is located in the housing 102 of the system 100. The oxygen concentrator 104 is controlled by the controller 116 of the system 100 but may alternatively be controlled by a separate controller (e.g., it is controlled by one or more controllers).

The oxygen concentrator 104 includes an inlet port 234, an outlet port 236, and a valve assembly 238. The inlet port 234 (e.g., an ambient air inlet port) is coupled to the inlet line 113 to obtain ambient air from the inlet 112 of the system 100. The oxygen concentrator 104 also includes an outlet port 236 (e.g., a product gas outlet port) that is coupled to the concentrator line 105 to supply the product gas to the supply port 110 through the concentrator line 105, the manifold 108, and the supply line 111. The inlet port 234 and the outlet port 236 may both be connected to the valve assembly 238 or otherwise connected to components of the oxygen concentrator 104.

The valve assembly 238 includes multiple controllable valves that are configured to regulate the flow of air and product gas between components of the oxygen concentrator 104. As an example, the valve assembly 238 may include solenoid valves that are controllable by the controller 116 (e.g., by supply of electrical signals to the solenoid valves). Other types of valves may be used. The valve assembly 238 is pneumatically connected to the inlet port 234, the outlet port 236, and to other components of the oxygen concentrator 104.

The oxygen concentrator 104 also includes a compressor 240 and at least one media bed containing an adsorbent material configured to separate oxygen from other components of the ambient air, such as media beds 242. To facilitate the oxygen concentration cycle, the oxygen concentrator 104 may also include a compressed air storage tank 244 and a product gas storage tank 246. The valve assembly 238 may be pneumatically connected to the compressor 240, the media beds 242, the compressed air storage tank 244, and the product gas storage tank 246. It should be noted that the product gas storage tank 246 is intended for temporary storage of oxygen generated by the oxygen concentrator 104 while the process of oxygen concentration is ongoing. Storage of oxygen by the product gas storage tank 246 is at much lower pressures than the oxygen supply tank 106, and the total volume of oxygen stored by the product gas storage tank 246 is much less than that stored by the oxygen supply tank 106. Stated differently, the product gas storage tank 246 is configured for use as a buffer to allow for oxygen supply at a stable rate and is not configured for high-pressure or high-volume storage of oxygen.

The compressor 240 is configured to compress air. The compressor 240 may be an air compressor configured to receive air from the inlet port 234 and increase the pressure of the air for use in oxygen concentration or for delivery as all or part of the product gas. The compressor 240 may be of any suitable type, such as a reciprocating compressor, a wobble compressor, a vane compressor, or a screw compressor. The compressor 240 may be configured to deliver the compressed air, as examples, to the outlet port 236, the media beds 242, and/or the compressed air storage tank 244. The compressed air may be delivered through the valve assembly 238 to control the destination of the compressed air under direction from the controller 116.

The media beds 242 include an adsorbent material that is contained within a pressurizable vessel. The media beds 242 may be referred to as sieve modules or sieve beds. The adsorbent material of the media beds 242 is used to separate oxygen from other components of ambient air, such as nitrogen, through pressurization of the adsorbent material according to the PSA cycle or the VPSA cycle. As an example, the adsorbent material may be a zeolite material. Thus, the oxygen concentrator 104 may include adsorbent media beds, such as the media beds 242, that receive compressed air from the compressor 240 and are controlled according to at least one of the PSA cycle or the VPSA cycle to generate the product gas, such as oxygen-enriched gas. In a typical implementation, two of the media beds 242 are included and are controlled separately so that one of the media beds 242 is always available to supply oxygen. For example, a first one of the media beds 242 may be supplying oxygen while a second one of the media beds 242 is in the nitrogen purge phase of its cycle. Thus, in some implementations, the oxygen concentrator 104 operates according to at least one of a pressure swing adsorption cycle or a vacuum pressure swing adsorption cycle using the media beds 242 for nitrogen adsorption.

The compressed air storage tank 244 is configured to store a small volume of air subsequent to compression thereof by the compressor 240 and prior to introduction of the compressed air into the media beds 242. The compressed air storage tank 244 may be used, for example, to help control delivery of compressed air to the media beds 242 at an appropriate time during the oxygen concentration cycle and at an appropriate pressure. The product gas storage tank 246 is configured to store a small volume of product gas (e.g., substantially pure oxygen) subsequent to release of the product gas from the media beds 242 and prior to delivery of the product gas via the outlet port 236 and the concentrator line 105. Thus, the product gas storage tank 246 may allow for a steady supply of oxygen by the oxygen concentrator 104 despite the fact that the oxygen concentrator 104 is operating using a cycle (e.g., the PSA cycle or the VPSA cycle) through which oxygen is released from the media beds 242 cyclically as opposed to continuously.

To produce the product gas (e.g., oxygen-enriched gas) using the media beds 242, air from the inlet port 234 is supplied to the compressor 240 by operation of the valve assembly 238. The pressurized air is then supplied from the compressor 240 or from the compressed air storage tank 244 to the media beds 242 at a pressure that is higher than ambient pressure. The air is subsequently released from the media beds 242 as the product gas. In particular, after the media beds 242 are pressurized, the valve assembly 238 is operated to establish fluid communication between the media beds 242 and the outlet port 236, in order to supply the product gas to the outlet port 236 for use.

The pressure at which the air is supplied to the media beds 242 is selected based on the material properties of the adsorbent material in the media beds 242. In particular, adsorption is dependent on pressure, and the volume of a gas that is adsorbed by the adsorbent material increases as the pressure rises. In addition, nitrogen is adsorbed by the adsorbent material that is used in the media beds 242 (e.g., zeolite) more readily than oxygen is at the same pressure. Thus, pressurization of the media beds 242 causes adsorption of a portion of the nitrogen in the air supplied to the media beds 242, and oxygen can be released from the media beds 242 while the nitrogen is retained in the media beds 242 by the adsorbent material. The oxygen supplied from the media beds 242 may be stored in the product gas storage tank 246 and/or supplied for use as the product gas through the outlet port 236. As needed, the media beds 242 can be purged by lowering the pressure in the media beds 242 so that the nitrogen is released from the adsorbent material, and this gas is vented from the oxygen concentrator 104, for example, through a vent port 248 that is connected to the vent line 115 and the vent 114.

Operation of the oxygen concentrator 104 is regulated by the controller 116 and the interface device 118. The controller 116 is configured to control operation of the oxygen concentrator 104 and components thereof (e.g., the compressor 240, the valve assembly 238, and/or other components) by electrical connections of the controller 116 to the components in order to cause operation of the components according to outputs from the controller 116. The controller 116 may include a general-purpose computing device, such as a computing device that includes one or more processors, a short-term memory device, and a long-term storage device. In some embodiments, the controller 116 may include a special-purpose computing device, such as an integrated circuit or an application-specific integrated circuit. The controller 116 may be provided with control software that is executed by the controller 116 to cause the controller 116 to operate the various components of the oxygen concentrator 104 in the desired manner. The interface device 118 is in electrical communication with the controller 116, and may include components such as buttons, knobs, and other types of input components that allow a user to change the operating state of the oxygen concentrator 104, such as by starting and stopping production of the product gas. The interface device 118 may include output components that show information regarding the state of the oxygen concentrator 104. The interface device 118 may be communicatively coupled to the controller 116, whereby the controller 116 receives user inputs from the interface device 118 and transmits operational status information to the interface device 118 for display to the user. As used herein, "communicatively coupled" refers to a connection between components that enables the transmission of signals, data, or control information, and includes electrical connections, wired connections, wireless connections, or combinations thereof.

The controller 116 is configured to control the manifold 108 to change the source of the oxygen that is output by the system 100. In some implementations, the controller 116 is configured to control the manifold 108 to selectively provide oxygen to the supply port 110 from the oxygen concentrator 104 only, from the oxygen supply tank 106 only, or from the oxygen concentrator 104 and the oxygen supply tank 106 simultaneously.

In some implementations, the controller 116 is configured to monitor pressure levels in both the oxygen concentrator 104 and the oxygen supply tank 106 using sensors that are incorporated in the system 100. The sensors may include at least one pressure sensor 128 configured to measure a pressure level within the oxygen supply tank 106. The controller 116 is configured to detect a depletion condition of the oxygen supply tank 106 based on a detected pressure level measured by the at least one pressure sensor 128. The depletion condition may correspond to a state in which the detected pressure level within the oxygen supply tank 106 falls below a predetermined threshold value, indicating that the oxygen supply tank 106 has insufficient oxygen remaining for continued use at a desired flow rate. The predetermined threshold value may be set during manufacture or configuration of the system 100 and represents a minimum pressure level below which continued reliable oxygen delivery from the oxygen supply tank 106 may not be assured. Using information from the at least one pressure sensor 128, the controller 116 is configured to generate an alert when the oxygen supply tank 106 requires replacement or refilling, such as when the detected pressure level in the oxygen supply tank 106 falls below the predetermined threshold value. The alert may be a visual or audible alert that is output using the interface device 118.

In some implementations, the controller 116 distinguishes between an alert condition and a depletion condition using two separate predetermined threshold values. The alert condition corresponds to a first predetermined threshold value at which the pressure level within the oxygen supply tank 106 has fallen sufficiently to warrant notifying the user that replacement or refilling of the oxygen supply tank 106 may soon be required, but at which continued delivery of oxygen from the oxygen supply tank 106 remains possible. The depletion condition corresponds to a second predetermined threshold value that is lower than the first predetermined threshold value and indicates that the oxygen supply tank 106 has insufficient oxygen remaining for continued reliable delivery at the desired flow rate. Upon detecting that the pressure level within the oxygen supply tank 106 has fallen below the first predetermined threshold value, the controller 116 generates an alert through the interface device 118. Upon detecting that the pressure level has subsequently fallen below the second predetermined threshold value, the controller 116 determines that the depletion condition has been met and automatically transitions the manifold 108 to the second state to deliver oxygen solely from the oxygen concentrator 104. In this manner, the user receives advance notice of impending tank depletion before the automatic switchover occurs, allowing time to replace or refill the oxygen supply tank 106 while oxygen delivery continues uninterrupted.

The system 100 allows for the supply of oxygen from the supply port 110 to be switched between supply from the oxygen supply tank 106 and the oxygen concentrator 104. This dual-source operation allows for flexible and efficient oxygen delivery. In addition, supply from the oxygen supply tank 106 may occur at rates higher than those achievable using the oxygen concentrator 104. The source of the oxygen provided by the system 100 may be selected using the interface device 118. In some implementations, the controller 116 is further configured to allow a user to indicate a user-indicated oxygen flow rate through the interface device 118. The user-indicated flow rate is the flow rate specified by the user via the interface device 118, and the controller 116 adjusts operation of the oxygen concentrator 104 and the manifold 108 based on the user-indicated flow rate. The controller 116 may automatically select among the first state, the second state, and the combined state of the manifold 108 based on the user-indicated flow rate or based on a detected pressure level in the oxygen supply tank 106. Thus, the source of the oxygen provided by the system 100 may be automatically selected by the controller 116 based on the user-indicated flow rate or based on another user input.

In an example implementation, the oxygen concentrator 104 may be configured to deliver oxygen at rates less than or equal to a first flow rate, such as four liters per minute, ensuring continuous availability without reliance on stored oxygen. When higher oxygen flow rates (e.g., at a second flow rate greater than four liters per minute) are required, the system transitions to use of the oxygen supply tank 106. The oxygen supply tank 106 may be configured to deliver oxygen via the system 100 at a flow rate of fifteen liters per minute. The flow rate provided by the system 100 may be controlled using the interface device 118.

In some implementations, the controller 116 is configured to automatically select an operating mode of the manifold 108 based on a flow rate selected by a user through the interface device 118. The flow rate selected by the user through the interface device 118 may be referred to as a user-selected oxygen flow rate. The controller 116 adjusts operation of the manifold 108 and the oxygen concentrator 104 based on the user-selected oxygen flow rate to meet the oxygen delivery needs of the user. When the user-selected oxygen flow rate is within a range achievable by the oxygen concentrator 104 alone (e.g., up to four liters per minute), the controller 116 controls the manifold 108 to supply oxygen solely from the oxygen concentrator 104. When the user-selected oxygen flow rate exceeds the maximum flow rate achievable by the oxygen concentrator 104 alone, the controller 116 controls the manifold 108 to supply oxygen from the oxygen supply tank 106, from the oxygen concentrator 104 and the oxygen supply tank 106 simultaneously, or a combination thereof, in order to meet the user-selected oxygen flow rate.

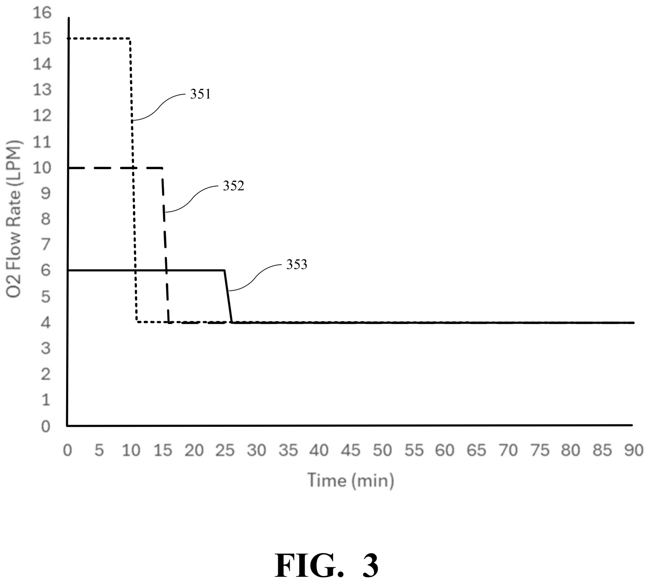

FIG. 3 is a graph that shows oxygen flow rates over time in a first scenario 351, a second scenario 352, and a third scenario 353, each of which represents a short-term supply of oxygen at an increased flow rate from the oxygen supply tank 106, and subsequent supply of oxygen at a lower flow rate using the oxygen concentrator 104. In the first scenario 351, oxygen is supplied from the oxygen supply tank 106 at a rate of fifteen liters per minute for ten minutes, at which point the oxygen supply tank 106 is depleted and the controller 116, sensing depletion of the oxygen supply tank 106, switches the supply mode to supply oxygen from the oxygen concentrator 104 at a lower rate, such as four liters per minute. Thus, the controller 116 may be configured to detect depletion of oxygen in the oxygen supply tank 106 and automatically switch the manifold 108 to deliver oxygen solely from the oxygen concentrator 104. In the second scenario 352, oxygen is supplied from the oxygen supply tank 106 at a rate of ten liters per minute for fifteen minutes, at which point the oxygen supply tank 106 is depleted and the controller 116, sensing depletion of the oxygen supply tank 106, switches the supply mode to supply oxygen from the oxygen concentrator 104 at a lower rate, such as four liters per minute. In the third scenario 353, oxygen is supplied from the oxygen supply tank 106 at a rate of six liters per minute for twenty-six minutes, at which point the oxygen supply tank 106 is depleted and the controller 116, sensing depletion of the oxygen supply tank 106, switches the supply mode to supply oxygen from the oxygen concentrator 104 at a lower rate, such as four liters per minute.

In another scenario, the controller 116 controls the manifold 108 to supply oxygen from the oxygen concentrator 104 and the oxygen supply tank 106 concurrently. As an example, for a period of twenty-six minutes, the oxygen concentrator 104 may supply four liters per minute, which is combined with a supply of six liters per minute from the oxygen supply tank 106, resulting in a supply of ten liters per minute for twenty-six minutes, at which point the oxygen supply tank 106 is depleted and the controller 116, sensing depletion of the oxygen supply tank 106, switches the supply mode to supply oxygen from only the oxygen concentrator 104 at a lower rate, such as four liters per minute.

Thus, in some implementations, the oxygen supply tank 106 is configured to provide oxygen at a first flow rate for a first time period, the oxygen concentrator 104 is configured to provide oxygen at a second flow rate for a second time period, the first flow rate is higher than the second flow rate, and the second time period is longer than the first time period.

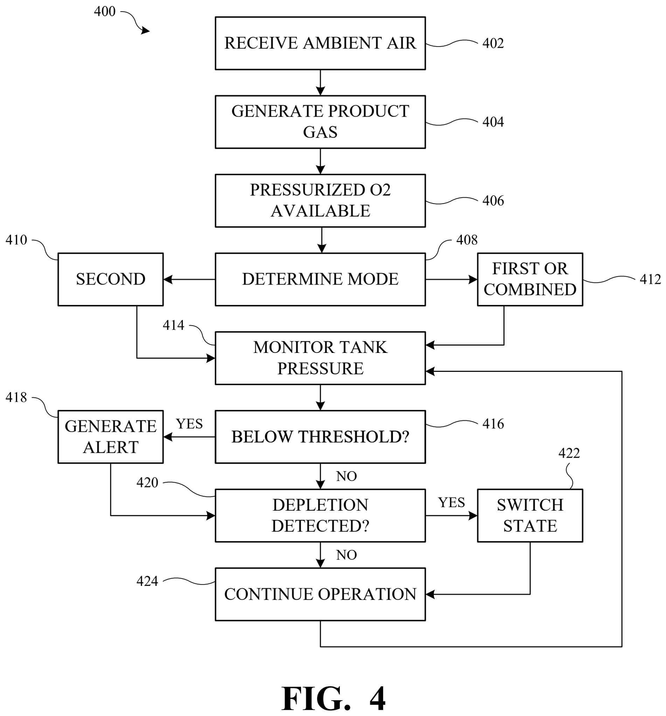

FIG. 4 is a flowchart of a process 400 of operating a portable multiple-source oxygen supply system, such as the system 100. The process 400 begins at operation 402, in which the oxygen concentrator 104 receives ambient air through the inlet 112 via the inlet line 113. At operation 404, the oxygen concentrator 104 generates a product gas comprising substantially pure oxygen by separating oxygen from other components of the ambient air using the media beds 242 containing an adsorbent material, such as zeolite. The product gas is made available to the manifold 108 via the concentrator line 105. Concurrently, at operation 406, pressurized oxygen stored in the oxygen supply tank 106 is made available to the manifold 108 via the tank line 127, the system-side coupler 126, and the tank-side coupler 124.

At operation 408, the controller 116 determines the appropriate operating mode for the manifold 108. In one implementation, the controller 116 receives a user-selected oxygen flow rate through the interface device 118 and evaluates whether the user-selected flow rate is within the output capacity of the oxygen concentrator 104 alone. If the user-selected flow rate is within the range achievable by the oxygen concentrator 104 alone, the controller 116 configures the manifold 108 in the second state at operation 410, routing oxygen from the oxygen concentrator 104 to the supply port 110 via the supply line 111. If the user-selected flow rate exceeds the maximum flow rate achievable by the oxygen concentrator 104 alone, the controller 116 configures the manifold 108 in the first state or the combined state at operation 412, routing oxygen from the oxygen supply tank 106 alone or from both the oxygen supply tank 106 and the oxygen concentrator 104 simultaneously to the supply port 110 to meet the user-selected flow rate.

At operation 414, the controller 116 monitors a pressure level within the oxygen supply tank 106 using the at least one pressure sensor 128 located on the tank line 127. At operation 416, the controller 116 determines whether the monitored pressure level has fallen below a predetermined threshold value. If the pressure level remains at or above the predetermined threshold value, the process 400 returns to operation 414 to continue monitoring. If the pressure level falls below the predetermined threshold value, the controller 116 proceeds to operation 418 and generates an alert through the interface device 118 to notify the user that the oxygen supply tank 106 requires replacement or refilling. The alert may be a visual alert, an audible alert, or a combination thereof output by the interface device 118.

At operation 420, the controller 116 determines whether a depletion condition of the oxygen supply tank 106 has been detected. A depletion condition may be indicated by the pressure level within the oxygen supply tank 106 falling below a second, lower predetermined threshold value, or by another depletion indicator recognized by the controller 116. If no depletion condition is detected, the process 400 continues in the current operating state. Upon detection of the depletion condition, the controller 116 automatically transitions the manifold 108 to the second state at operation 422, routing oxygen to the supply port 110 solely from the oxygen concentrator 104, thereby maintaining continuous oxygen delivery to the patient notwithstanding depletion of the oxygen supply tank 106.

At operation 424, the oxygen concentrator 104 generates the product gas by operating according to at least one of a pressure swing adsorption (PSA) cycle or a vacuum pressure swing adsorption (VPSA) cycle using the media beds 242. In the PSA cycle, ambient air drawn through the inlet 112 is pressurized by the compressor 240 and directed through the media beds 242, which contain an adsorbent material that selectively adsorbs nitrogen, yielding an oxygen-enriched product gas. In the VPSA cycle, the media beds 242 are additionally subjected to a vacuum during the purge phase to more completely desorb the retained nitrogen, which is vented through the vent port 248 and the vent 114. The product gas is directed from the media beds 242 to the product gas storage tank 246 and subsequently delivered via the outlet port 236 and the concentrator line 105 to the manifold 108 and the supply port 110, either alone in the second state or in combination with pressurized oxygen from the oxygen supply tank 106 in the combined state. The process 400 thereafter continues with the controller 116 monitoring operating conditions and adjusting the operating mode of the manifold 108 as required to maintain the prescribed oxygen delivery.

The above-described aspects, examples, and implementations have been described in order to allow easy understanding of the disclosure and are not limiting. On the contrary, the disclosure covers various modifications and equivalent arrangements included within the scope of the appended claims, which scope is to be accorded the broadest interpretation so as to encompass all such modifications and equivalent structures as is permitted under the law.

When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

Protection may be sought for any features disclosed in any one or more published documents referenced herein in combination with the present disclosure.

Although certain example embodiments of the invention have been described, the scope of the appended claims is not intended to be limited solely to these embodiments. The claims are to be construed literally, purposively, and/or to encompass equivalents.

## Claims

1. A portable multiple-source oxygen supply system comprising:
a housing;
a portable oxygen concentrator located in the housing and configured to receive ambient air and generate substantially pure oxygen as a product gas, the portable oxygen concentrator including at least one media bed containing an adsorbent material configured to separate oxygen from other components of the ambient air, a compressor configured to pressurize the ambient air, and a valve assembly configured to regulate flow of the ambient air and the product gas;
an oxygen supply tank configured to store pressurized oxygen, wherein the oxygen supply tank is removably coupled to the housing, and the oxygen supply tank is refillable and replaceable;
a manifold located in the housing and in fluid communication with the portable oxygen concentrator and the oxygen supply tank, the manifold configured to selectively route oxygen from the portable oxygen concentrator and the oxygen supply tank to a supply port; and
a controller operably located in the housing and electrically connected to the manifold and the portable oxygen concentrator, the controller configured to control operation of the portable oxygen concentrator to generate the product gas, and to control the manifold to selectively provide oxygen to the supply port from the portable oxygen concentrator only, from the oxygen supply tank only, or from the portable oxygen concentrator and the oxygen supply tank simultaneously.

2. The portable multiple-source oxygen supply system of claim 1, wherein the oxygen supply tank comprises:
a pressure vessel configured to store the pressurized oxygen, and
a tank-side coupler configured to selectively connect and disconnect the oxygen supply tank from the manifold using a system-side coupler.

3. The portable multiple-source oxygen supply system of any preceding claim, wherein the manifold comprises at least one electrically operated valve operable between a first state routing oxygen from the oxygen supply tank to the supply port and a second state routing oxygen from the portable oxygen concentrator to the supply port.

4. The portable multiple-source oxygen supply system of any preceding claim, wherein the controller is configured to detect depletion of oxygen in the oxygen supply tank and automatically switch the manifold to deliver oxygen solely from the portable oxygen concentrator.

5. The portable multiple-source oxygen supply system of any preceding claim, wherein the manifold further includes a combined supply mode configured to mix oxygen from the portable oxygen concentrator with oxygen from the oxygen supply tank to achieve an increased flow rate at the supply port.

6. The portable multiple-source oxygen supply system of any preceding claim, further comprising:
a power source that provides electrical power to the portable oxygen concentrator, the controller, and the manifold.

7. The portable multiple-source oxygen supply system of any preceding claim, wherein the controller is further configured to allow a user to indicate a selected oxygen flow rate through an interface device, and wherein the controller adjusts operation of the portable oxygen concentrator and the manifold based on the selected oxygen flow rate.

8. The portable multiple-source oxygen supply system of any preceding claim, wherein the portable oxygen concentrator operates according to at least one of a pressure swing adsorption cycle or a vacuum pressure swing adsorption cycle using the at least one media bed for nitrogen adsorption.

9. The portable multiple-source oxygen supply system of any preceding claim, wherein an interface device provides user-accessible controls for activating the portable oxygen concentrator, selecting flow rates, and displaying system status.

10. The portable multiple-source oxygen supply system of any preceding claim, wherein the oxygen supply tank is configured to provide oxygen at a first flow rate for a first time period, the portable oxygen concentrator is configured to provide oxygen at a second flow rate for a second time period, the first flow rate is higher than the second flow rate, and the second time period is longer than the first time period.

11. The portable multiple-source oxygen supply system of any preceding claim, wherein the controller is further configured to monitor pressure levels in both the portable oxygen concentrator and the oxygen supply tank to generate an alert when the oxygen supply tank requires replacement or refilling.

12. The portable multiple-source oxygen supply system of any preceding claim, wherein the oxygen supply tank is located external to the housing.

13. A method of operating a portable multiple-source oxygen supply system, the method comprising:
receiving ambient air at a portable oxygen concentrator housed within a housing;
generating, by the portable oxygen concentrator, a product gas comprising substantially pure oxygen by separating oxygen from other components of the ambient air using at least one media bed containing an adsorbent material;
providing pressurized oxygen in an oxygen supply tank that is removably coupled to the housing;
controlling, by a controller, a manifold in fluid communication with the portable oxygen concentrator and the oxygen supply tank to selectively route oxygen to a supply port from the portable oxygen concentrator only, from the oxygen supply tank only, or from the portable oxygen concentrator and the oxygen supply tank simultaneously; and
detecting, by the controller, a depletion condition of the oxygen supply tank and automatically switching the manifold to deliver oxygen solely from the portable oxygen concentrator in response to detection of the depletion condition.

14. The method of claim 13, further comprising:
receiving, by the controller, a user-selected oxygen flow rate through an interface device; and
adjusting, by the controller, operation of the portable oxygen concentrator and the manifold based on the user-selected oxygen flow rate, including transitioning to supply from at least the oxygen supply tank when the user-selected oxygen flow rate exceeds a maximum flow rate achievable by the portable oxygen concentrator alone.

15. The method of claim 13 or claim 14, further comprising:
monitoring, by the controller, a pressure level within the oxygen supply tank using at least one pressure sensor; and
generating an alert through an interface device when the pressure level falls below a predetermined threshold value indicating that the oxygen supply tank requires at least one of replacement or refilling.
